Europäisches Patentamt

European Patent Office

Office européen des brevets

(11) Publication number: **0 022 432**
**B1**

(12) **EUROPEAN PATENT SPECIFICATION**

(45) Date of publication of patent specification: **17.10.84**

(51) Int. Cl.³: **C 12 Q 1/50, C 12 Q 1/66**

(21) Application number: **80850093.8**

(22) Date of filing: **17.06.80**

(54) Method for determination of creatine kinase.

(30) Priority: **04.07.79 SE 7905852**

(43) Date of publication of application:
**14.01.81 Bulletin 81/02**

(45) Publication of the grant of the patent:
**17.10.84 Bulletin 84/42**

(84) Designated Contracting States:
**BE DE FR GB NL**

(56) References cited:
GB-A-1 163 409
GB-A-2 005 830
US-A-4 001 088
US-A-4 080 265
US-A-4 097 338

HANS ULRICH BERGMEYER: "Methoden der curymetischen Analyse", vol. 2, Verlag Chemie, 1974 Weinheim, DE STREHLER B.L. "Ademosin-5'-Triphosphat und Creatinphosphat. Bestimmung mit Luciferase", pages 2163-2177

(73) Proprietor: **LKB-Produkter AB**
**Box 305**
**S-161 26 Bromma (SE)**

(72) Inventor: **Lundin, Arne Thorwald**
**Gaveliusgatan 6**
**S-116 41 Stockholm (SE)**
Inventor: **Lövgren, Timo**
**Regementsvägen 64 I 119**
**SF-20 880 Turku 88 (FI)**

(56) References cited:
CHEMICAL ABSTRACTS, vol. 89 no. 17, October 23, 1978, page 229, Abstract 142374m, Columbus, Ohio, USA LUNDIN, ARNE et al. "Sensitive essay of creatine kinase isoenzymes in human serum using M subunit inhibiting antibody and firefly luciferase"
CHEMICAL ABSTRACTS, vol. 92, no. 5, February 4, 1980, abstract no. 36513h, page 314. Columbus, Ohio, USA DEBTON MARK S. et al. "Continuously referenced, on line monitoring of creatine kinase and lactate dehydrogenase isoenzymes for use in clinical diagnostics"

Courier Press, Leamington Spa, England.

**0 022 432**

(56) References cited:
CLINICAL CHEMISTRY, vol. 18, no. 5, 1972, pages 473-475. WILLIAM A. WARREN "Activation of serum creatine kinase by dithiothreitol"
CLINICA CHIMICA ACTA, vol. 40, 1972, pages 133-138. HANNU SOMER et al. "Demonstration of serum creatine kinase isoenzymes by fluorescence technique"

## Description

The present invention refers to a method for determination of creatine kinase. More specifically, the invention refers to a technique in which the enzyme creatine kinase and its two substrates ADP and creatine phosphate are brought in contact with a bioluminescence reagent having a stable light emission and comprising firefly luciferase, D-luciferin and certain metal ions, such as magnesium ions, whereafter the light emission is measured. The bioluminescence reagents having a stable light emission proportional to the ATP concentration are described by Lundin and Myhrman in the Swedish Patent Application 7806296—5. The concentration of the ATP formed by the creatine kinase reaction can be continuously monitored by a bioluminescence reagent of this type and the creatine kinase activity can be measured as the rate of increase of the light emission. The reaction formulas are as follows:

$$\text{ADP} + \text{creatine phosphate} \xrightarrow{\text{creatine kinase}} \text{ATP} + \text{creatine}$$

$$\text{ATP} + \text{D-luciferin} + O_2 \xrightarrow{\text{luciferase}} \text{AMP} + \text{pyrophosphate} + \text{oxiluciferin} + CO_2 + \text{light}$$

The hitherto used method for the above determination has been elaborated by Lundin and Styrelius (Clin. Chim. Acta 87 (1978) 199). The bioluminescence reagent then used had analytical properties similar to those described in the Swedish Patent Application No. 7806296—5 but was in certain respects badly controlled and partly unspecified and was furthermore not possible to produce routinely. The method described by Lundin and Styrelius did however appear to be much more sensitive than the corresponding spectrophotometric method (see the comparison in the above reference by Lundin and Styrelius). The sensitivity was even adequate for determination of the subunit B of creatine kinase, which activity is more cardial specific than a determination of the total creatine kinase activity, also in serum from healthy people. Results showing this have been published by Lundin, Lindberg, Nordlander, Nyquist and Styrelius (Publication in press, presented at the International Symposium on Analytical Applications of Bioluminescence and Chemiluminescence, Brussels, September 1978). This is of big clinical interest since it makes possible to discover also small myocardial infarctions during an early stage of the infarction process.

The method described by Lundin and Styrelius has however been less suited for clinical routine use depending partly on the fact that the bioluminescence reagent used by Lundin and Styrelius has not been possible to produce routinely, partly because of the fact that ADP concentrations saturating the creatine kinase reaction have not been possible to use. The background of the first disadvantage is described in the Swedish Patent Application No. 7806296—5. The reason why a saturating ADP concentration has not been possible to use has been that the forming of ATP through the adenylate kinase reaction, which disturbs the analysis, at an increase of the ADP concentration increases more than the creatine kinase activity. The result is consequently that low creatine kinase activities are not possible to detect due to the adenylate kinase activity. This problem is described by Lundin and Styrelius and by Lundin and cooperators in the above references. The disadvantages of not using non-saturating ADP concentrations in the determination is firstly that the creatine kinase activity expressed as the formation of ATP per unit of time is lower than in the conventional spectrophotometric analysis which is confusing for the operator, secondly that the creatine kinase activity is dependent upon the ADP concentration, which means that small errors in this concentration directly results in an error of the determination of creatine kinase.

In the less sensitive spectrophotometric determination of creatine kinase, the adenylate kinase is inhibited by millimolar concentrations of AMP. Lundin and cooperators (reference see above) tried to use this method for inhibiting the adenylate kinase in the bioluminescence method but found that the inhibition of the luciferase reaction was approximately just as big as the inhibition of the adenylate kinase (already a concentration of 0.1 mM AMP gave rise to an inhibition of approximately 50%). The inhibition of the luciferase reaction was disturbing mainly due to the fact that it was dependent upon the concentration. Thus, a small error of the AMP concentration would change the luciferase activity and give rise to an error at the determination of the creatine kinase activity. Even if the addition of AMP made it possible to use a saturating concentration of ADP which in fact was never shown, the determination of creatine kinase would still be dependent upon the concentration of the AMP.

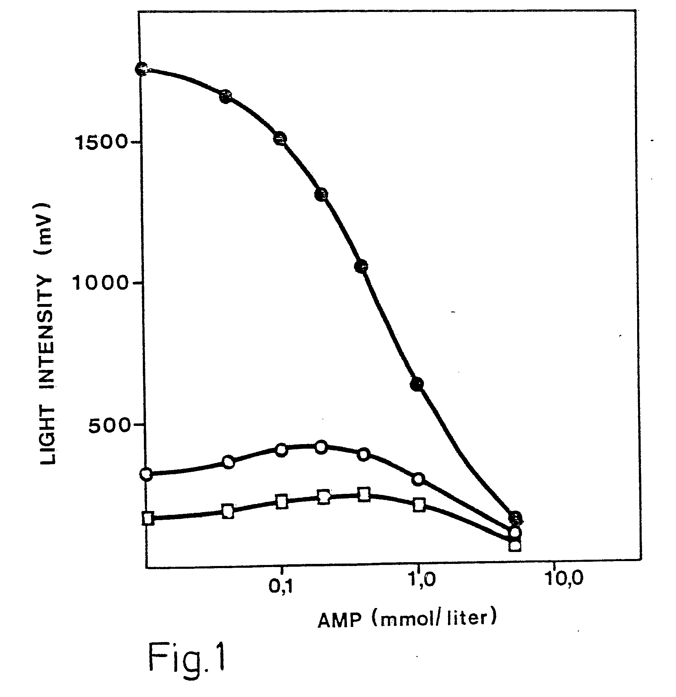

According to the invention it has been shown that AMP also in rather high concentrations can be added without inhibiting the light emission of a bioluminescence reagent having a stable light emission provided that the determinations are performed at a pH value around or just below 7. This is shown in Fig. 1 which is a diagram showing the light emission as a function of the AMP concentration. In Fig. 1 the upper curve shows the situation at a pH value of 7.5, the next curve shows the situation at a pH value of 6.7 and the lowest curve shows the situation at a pH value of 6.5. The reaction mixture (1.0 ml) comprised 200 $\mu$l bioluminescence reagent having a stable light emission and being produced in accordance with the Swedish Patent Application No. 7806296—5, 0.01 mol/l creatine phosphate

and the above defined quantities of AMP.

Even if the invention is not limited to any specific theory concerning the reaction mechanism enabling an addition of AMP in concentrations which significantly inhibit the adenylate kinase reaction without any significant effect on the light emission from the luciferase reaction detected, the most likely reason is that this addition activates the light emission in the yellow green wavelength range simultaneously as the red emission is inhibited. Lee and McElroy (Arch. Biochem. Biophys. 145 (1971) 78) have described a similar spectral displacement of the light emission under conditions which do not give stable light emission. Since an important part of the light emission is constituted by red light under the conditions present at the determination of creatine kinase (pH 6.7) one should in order to decrease the inhibition of the light detected choose a light detector which has a high ratio for the sensitivity of yellow green and the sensitivity of red light. The higher AMP concentrations one wishes to add the higher this ratio has to be in order to compensate the inhibition of the red light emission by the activation of the yellow green emission. In very high AMP concentrations also the yellow green light emission is inhibited and such AMP concentrations can consequently not be added. Furthermore, it is according to Lee and McElroy (reference see above) possible that the activation of the yellow green light emission at an addition of AMP is due to the fact that luciferase changes its conformation. It is consequently important to choose such conditions for the analysis that this change of conformation can take place.

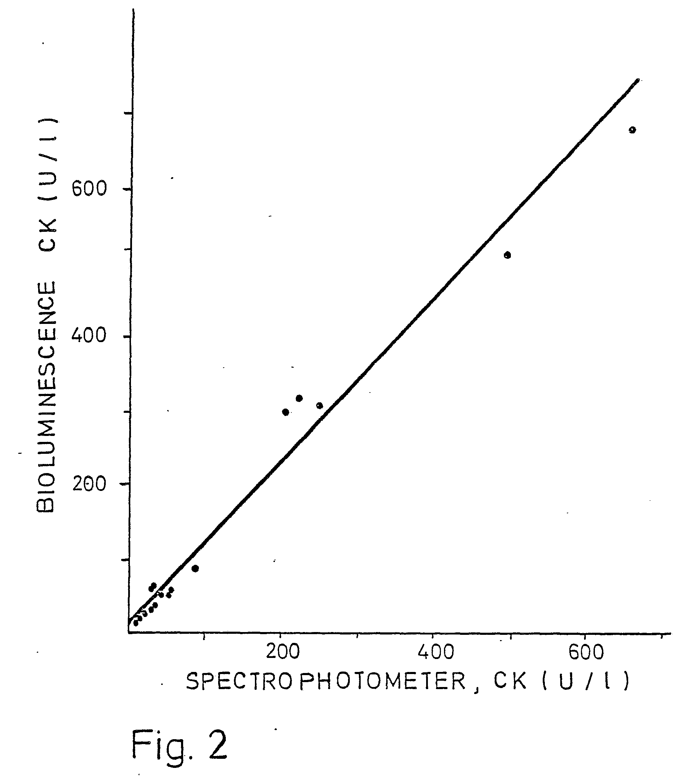

The advantages of determining creatine kinase in the presence of AMP according to the invention are important as compared to techniques known per se. The addition of AMP implies that the adenylate kinase activity can be inhibited in order to make possible to determine the activity of the subunit B of creatine kinase in serum from healthy people. In addition to the determination of the creatine kinase activity independently of smaller variations of the ADP concentration the method according to the invention makes it possible to express the activity of the formation of ADP per time unit just as big as according to the spectrophotometric routine method as shown in Fig. 2 which is a diagram showing the correlation between the spectrophotometric and the bioluminescence method for determining of creatine kinase in a serum sample. This is important when the results are to be evaluated by the clinical chemist. A further advantage is that since the reaction rate of the creatine kinase reaction is higher, it is possible to use less serum for the determination. This is important since it is often difficult to obtain sufficient amounts of serum and furthermore a certain interference on the luciferase reaction was obtained from serum at the concentration (50 $\mu$l

per ml reaction mixture) used by Lundin and Styrelius (reference see above). At low concentrations of serum in the determination the luciferase activity is not affected by the addition of serum and it will not be necessary to determine this addition by adding a known amount of ATP for each sample. The analysis procedure is hereby considerably simplified. This consequence of the procedure according to the invention and the fact that the reaction is higher will imply that the measuring time for the analysis is considerably reduced (less than 30 seconds for determining of the total creatine kinase activity). This is an important advantage both compared to the previously known bioluminescence determination of creatine kinase activity and the corresponding spectrophotometric method. This advantage is specifically important for instance when automatic analysers are used for the determination where the number of analyses performed per hour is important.

Thus, the technical advantages obtained according to the process of the invention in determining creatine kinase according to the bioluminescence method makes it possible to perform the analysis in clinical routine work and makes it possible to produce commercial kits for this purpose. The clinical and economical significance of this is realised from the fact that worldwide several tenths of million determinations of the creatine kinase activity are carried out per year. The determination according to the invention has through its higher sensitivity and simplicity compared to the present technique big possibilities to obtain a considerable part of this market.

The invention will now be illustrated with the following non-limiting example:

Example

This example shows the composition and use of a kit for determining creatine kinase in human serum. The number of determinations per kit is 50. The kit contains the following units (one of each):

ATP Monitoring Reagent (LKB-Wallac, article No. 1250—121);

ATP Standard (LKB-Wallac, article No. 1250—122);

ADP reagent (25 $\mu$mol ADP, 50 $\mu$mol AMP and 0.01 $\mu$mol diadenosinpentaphosphate);

CP reagent (500 $\mu$mol creatine phosphate);

CK buffer (45 ml buffer containing 0.11 M imidazol);

2.2 mM EDTA and 22 mM N-acetylcystein adjusted to pH 6.7 with acetic acid).

All these reagents except the buffer are present in freezed dried state. For determining of creatine kinase-MB the kit does also contain one bottle of anti-M antibodies from goat .(Merck, Darmstadt) in freezed dried state.

Before use the ATP Monitoring Reagent is dissolved in a CK buffer (the mixture having a volume of 45 ml is called CK reagent below), ATP Standard is dissolved in 10 ml distilled water, ADP reagent in 1 ml distilled water, CP reagent in 1 ml distilled water. When determining the creatine kinase-MB also the anti-M antibodies are dissolved in the mixture of ATP Monitoring Reagent and CK buffer (CK reagent).

In each series of samples also a reagent blank without any addition of serum is added and furthermore a control serum having a known creatine kinase activity is analysed. Hereby, 10 $\mu$l ATP Standard is added as an internal calibration in order to make it possible to transform the light intensity value into an ATP concentration value.

### Claim

Method for the determination of creatine kinase activity in for instance serum, whereby the sample to be determined is brought in contact with the two substrates of the creatine kinase reaction, ADP and creatine phosphate and a bioluminescence reagent having a light emission stable with respect to time based on firefly luciferase and D-luciferin, whereafter the rate of increase of the light emission is measured, which rate constitutes a measure of the creatine kinase activity, characterized in that AMP is added to a concentration less than $10^{-3}$ M so as to significantly inhibit the disturbing adenylate kinase activity without any significant affection of the light emission derived from the luciferase reaction.

### Patentanspruch

Verfahren zur Bestimmung der Kreatinkinaseaktivität in beispielsweise Serum, bei dem die zu untersuchende Probe mit den beiden Substraten der Kreatinkinasereaktion ADP und Kreatinphosphat und einem eine zeitlich stabile Lichtemission aufweisenden Bioluminiszenzreagens auf der Basis von Leuchtkäferluciferase und D-Luciferin in Berührung gebracht wird, wonach die ein Maß der Kreatinkinaseaktivität darstellende Geschwindigkeit des Anstiegs der Lichtemission gemessen wird, dadurch gekennzeichnet, daß zur erheblichen Hemmung der störenden Adenylatkinaseaktivität ohne erhebliche Auswirkung auf die Lichtemission aus der Luciferasereaktion AMP bis zu einer Konzentration von weniger als $10^{-3}$ M zugegeben wird.

### Revendication

Méthode de dosage de l'activité en créatine kinase par exemple dans du sérum, dans laquelle l'échantillon à doser est mis en contact avec les deux substrats de la réaction de la créatine kinase, ADP et phosphate de créatine, et avec un réactif de bioluminescence ayant une émission de lumière stable par rapport au temps basée sur la luciférase de luciole et la D-luciférine, après quoi on mesure la vitesse d'accroissement de l'émission de lumière, cette vitesse constituant une mesure de l'activité en créatine kinase, caractérisée en ce que de l'AMP est ajouté jusqu'à une concentration inférieure à $10^{-3}$ M de manière à inhiber notablement l'activité perturbatrice en adénylate kinase sans affecter sensiblement l'émission de lumière qui dérive de la réaction de la luciférase.

Fig.1

Fig. 2